(19) Europäisches Patentamt
European Patent Office
Office européen des brevets

(11) EP 0 650 376 B1

(12) EUROPÄISCHE PATENTSCHRIFT

(45) Veröffentlichungstag und Bekanntmachung des Hinweises auf die Patenterteilung:
25.09.1996 Patentblatt 1996/39

(51) Int. Cl.$^6$: **A61M 5/44**

(21) Anmeldenummer: 93914627.0

(22) Anmeldetag: 13.07.1993

(86) Internationale Anmeldenummer:
PCT/DE93/00624

(87) Internationale Veröffentlichungsnummer:
WO 94/02189 (03.02.1994 Gazette 1994/04)

(54) **VORRICHTUNG ZUM ERWÄRMEN VON FLÜSSIGKEITEN IM MEDIZINISCHEN ANWENDUNGSBEREICH**

DEVICE FOR HEATING LIQUIDS IN THE FIELD OF MEDICAL APPLICATIONS

DISPOSITIF VISANT A CHAUFFER DES LIQUIDES DANS LE DOMAINE D'APPLICATIONS MEDICALES

(84) Benannte Vertragsstaaten:
**AT CH DE FR GB IT LI NL**

(30) Priorität: **17.07.1992 DE 4223521**

(43) Veröffentlichungstag der Anmeldung:
**03.05.1995 Patentblatt 1995/18**

(73) Patentinhaber: **STIHLER ELECTRONIC MEDIZINTECHNISCHE GERÄTE PRODUKTIONS- UND VERTRIEBS- GMBH**
**D-70597 Stuttgart (DE)**

(72) Erfinder:
• **STIHLER, Axel**
**D-70839 Gerlingen (DE)**
• **THEILACKER-BECK, Wolfgang**
**D-70597 Stuttgart (DE)**

(74) Vertreter: **KOHLER SCHMID + PARTNER Patentanwälte Ruppmannstrasse 27 70565 Stuttgart (DE)**

(56) Entgegenhaltungen:
**EP-A- 0 181 447        DE-A- 3 606 930**
**DE-U- 9 209 604**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft eine medizinische Erwärmvorrichtung von Infusions-, Transfusions- und/oder Spüllösungen, mit einer an einer Leitung für die Lösungen wirksamen regel- und/oder steuerbaren Heizeinrichtung, die einen zylindrischen Wärmeaustauschkörper erwärmt, der auf der Außenseite zum Einlegen der von der Lösung durchströmten Leitung eine Nut aufweist, die Windungen mit einer Wärmeübertragungslänge I pro Windung bildet.

Eine derartige Vorrichtung ist durch die Druckschrift EP 0 181 447 B1 bekanntgeworden.

Die bekannten Geräte zum Erwärmen von Infusionen bzw. Transfusionen mit einem zylinderförmigen Wärmeaustauschkörper weisen auf der Außenseite eine schraubenlinienförmig verlaufende Nut auf, in die eine Leitung einlegbar ist, die von der zu erwärmenden Flüssigkeit durchströmt wird. Die in Gerätegehäusen gehaltenen Wärmeaustauschkörper sind aus einem gut wärmeleitfähigen Material gefertigt, damit über eine Kontaktwärmeübertragung die in der Leitung strömende Flüssigkeit erwärmt werden kann.

Bei dem bekannten Gerät nach EP 0 181 447 B1 ist der Wärmeaustauschkörper vertikal ausgerichtet und die im Außenbereich des Wärmeaustauschkörpers in einer Nut angeordnete Leitung ist von einer Wärmeschutzmanschette abgedeckt. Mittels einer an die Leitung angepaßten Nutform und durch die Abdeckung der in der Nut verlaufenden Leitung lassen sich die Wärmeabstrahlverluste gering halten und eine gleichmäßige Erwärmung der in der Leitung strömenden Flüssigkeit ist in einem hohen Maße gegeben.

Bei einem weiteren zum Stand der Technik zu zählenden Gerät, das durch die EP 0 444 011 A1 bekanntgeworden ist, ist der als gerader Kreiszylinder ausgeformte Wärmeaustauschkörper horizontal ausgerichtet und die die Flüssigkeit führende Leitung wird in eine auf der Außenseite des Kreiszylinders verlaufende Nut eingedrückt. Die Nut ist derart ausgestaltet, daß sie einerseits wesentlich tiefer ist als der Außendurchmesser der in die Nut einzulegenden Leitung und der Nutgrund ist zur Nutöffnung hin versetzt. Die Nut weist vom Nutgrund zur Nutöffnung verlaufende Seitenwände auf, die für die in die Nut einzulegende Leitung über die gesamte Nuttiefe eine Engstelle darstellen.

Neben den schon genannten Geräten ist weiterhin ein Gerät der Firma Dideco bekannt (siehe EP 0 181 447 B1 Spalte 1, Zeile 10 ff), das ebenfalls einen horizontal ausgerichteten Wärmeaustauschkörper aufweist, der als gerader Kreiszylinder ausgebildet ist.

Bei den bekannten Geräten ist die Wärmeübertragungslänge auf die die Flüssigkeit führende Leitung durch eine begrenzte Anzahl der Nutumschlingungen am Wärmeaustauschkörper limitiert. Werden wie bekannt die einzelnen Nutumschlingungen näher aneinander gelegt, so läßt sich die Wärmeübertragungslänge nur begrenzt verlängern und die Handhabung des Gerätes verschlechtert sich.

Bekannt sind Wärmeübertragungslängen bei zylindrisch ausgebildeten Wärmeaustauschkörpern bis zu ca. 37 cm pro Umschlingung (Windung).

Man muß weiterhin beachten, daß, ausgenommen beim Gerät nach EP 0 181 447 B1, auch neben der begrenzten Wärmeübertragungslänge noch eine unkontrollierte konvektive Wärmeabstrahlung im Außenbereich der bekannten Wärmeaustauschzylinder erfolgt, die den Wirkungsgrad einer effektiven Erwärmung der Flüssigkeit nicht verbessert. Werden bei den bekannten Wärmeaustauschzylindern die Nuten sehr tief ausgebildet, so besteht zusätzlich die Gefahr, daß die Stege zwischen den Nuten in einem weiten Bereich als Kühlrippen wirken und alle mit der jeweiligen Nutform verbundenen Vorteile aufheben können. Ebenfalls kann bei den bekannten tief ausgeführten Nutquerschnittsformen nicht mit einfachen Mitteln geprüft werden, ob die in die Nut eingelegte Leitung am Nutgrund formschlüssig anliegt. Ist dies nicht der Fall, so muß eine starke Minderung der Wärmeübertragung in Kauf genommen werden.

Der Erfindung ist deshalb die Problemstellung zugrunde gelegt, die bekannten Wärmeaustauschzylinder dahingehend weiterzubilden, daß die Abstrahlungsverluste zur Umgebung verringert werden.

Diese Aufgabe wird erfindungsgemäß dadurch gelöst, daß die Wärmeübertragungslänge I pro Windung mindestens 52 cm beträgt und daß die Nut im Bereich ihrer Öffnung aus einem wärmeisolierenden Material gefertigt oder mit einem wärmeisolierenaen Material beschichtet ist.

Ist die Nut im Bereich der Öffnung aus einem wärmeisolierenden Material gefertigt oder ist sie mit einem wärmeisolierten Material beschichtet, so kann die Nutform auch eine Tiefe aufweisen, die sehr viel größer ist als der Außendurchmesser der Leitung. Unerwünschten Kühlungseffekten der rippenartigen Stege der Nuten wird effektiv entgegengewirkt.

In einer Ausführungsform der Erfindung ist die Öffnung der Nut mindestens 1,6 mal größer als der Außendurchmesser der Leitung.

Dies hat den Vorteil, daß das Bedienungspersonal einfach durch Abtastung beispielsweise mit den Fingerkuppen überprüfen kann, ob die Leitung vollkommen in der Nut anliegt. Über die vergrößerte Nutöffnung kann die Leitung auch dann noch ertastet werden, wenn sie in einer vertieften Nut am Nutgrund anliegt. Ist dabei die Nut so ausgestaltet, daß sie sich bogenförmig öffnet und dabei einen ersten Nutraum mit Nutflahken zum Einlegen der Leitung bildet und sich daran ein zweiter Nutraum mit Nutflanken zum Halten der Leitung, eine Haltenut, anschließt, wobei die Nutflanken des ersten Nutraums konkav oder konvex oder trichterförmig verlaufen und die Nutflanken des zweiten Nutraumes die Leitung formschlüssig umgreifen und gegebenenfalls geringfügig verpressen, so ist neben einer erhöhten Bedienungsfreundlichkeit immer gewährleistet, daß die Leitung nicht nur teilweise am Nutgrund zur Anlage kommt.

Die erfindungs gemäße Wärmeübertragungslänge pro Windung ergibt einen umfangsmäßig großen Wärmeaustauschkörper.

Dies hat den Vorteil, daß sich die gesamte Wärmeübertragungslänge sogar bei reduzierter Nutumschlingungszahl um ein vielfaches verlängern läßt. Die Lange des Wärmeaustauschkörpers in Achsrichtung läßt sich gering halten und die einzelnen Nutumschlingungen können handhabungsgerecht voneinander beabstandet sein (große Steigungen), damit die Leitung einfach in die Nut einzulegen ist. Ein derartig umfangsmäßig großer Wärmeaustauschkörper ermöglicht es auch, daß alle Steuer-, Regelungs- und Bedienungskomponenten im Wärmeaustauschkörper selbst untergebracht werden können, d.h., wird der erfindungsgemäße Wärmeaustauschkörper horizontal ausgerichtet an einem Ständer befestigt, so ist er sehr viel sicherer dort befestigt als die bekannten Geräte. Das am Ständer wirkende Moment ist klein. Der Ständer mit dem daran aufgehängten Wärmeaustauschkörper ist somit sehr kippstabil. Werden bei den bekannten Geräten Leistungserhöhungen gewünscht, so müssen sie verlängert werden, und dies erzeugt im Normalfall größere Momente, die nur durch Gegenmaßnahmen (z.B. Gegengewichte) kompensiert werden können.

Bei dem erfindungsgemäßen Gerät sind solche zusätzlichen Sicherheitsvorkehrungen deshalb nicht notwendig, weil es aufgrund seiner Durchmessergröße sehr flach gebaut werden kann und das Gerätegewicht nahe an der Ständerstange wirkt.

Wird beim erfindungsgemäßen Gerät beispielsweise der Wärmeaustauschkörper als gerader Kreiszylinder mit einem Durchmesser von ca. 225 mm ausgeführt, so läßt sich die Wärmeübertragungslänge gegenüber den bekannten Geräten pro Nutumschlingung nahezu verdoppeln und die Tiefe des Gerätes nahezu halbieren. Dabei ist der Wärmeaustauschkörper horizontal ausgerichtet.

In einer bevorzugten Ausführungsform spannt der Wärmeaustauschkörper im Querschnitt zu einer Achse eine kreisförmige oder ellipsenähnliche Fläche auf.

Dies hat den Vorteil, daß der erfindungsgemäße Wärmeaustauschkörper mit einfachen Handbewegungen bedient werden kann. Die Leitung läßt sich sehr übersichtlich in die dafür vorgesehenen Nutwindungen einlegen und bei einer elliptischen Querschnittsfläche sieht der Benutzer ungehindert über einen weiten Anlagebereich sofort, wie und ob die Leitung, ein z. B. handeisübliches Infusionsbesteck, lagerichtig in der Nut gehalten ist.

Ist der Wärmeaustauschkörper als Hohlzylinder ausgeführt, so kann er neben der Heizungseinrichtung alle Bedienungselemente, und Kontrollelemente aufnehmen, ohne daß diese Konponenten aus dem Hohlzylinder herausragen.

In einer weiteren Ausgestaltung der Erfindung verläuft die Nut schraubenlinienförmig und die Nuten weisen im Querschnitt längs zu einer Achse des Wärmeaustauschkörpers gesehen eine Beabstandung voneinander auf, die größer als die Nutbreite einer Haltenut für die Leitung ist.

Dies hat den Vorteil, daß beim Aufwickeln der Leitung auf den Wärmeaustauschkörper eine Nutumschlingung nicht unbeabsichtigt übersprungen wird und dabei möglicherweise noch eine Abknickung der Leitung erfolgt. Die Leitung ist beim Aufwickeln auf den erfindungsgemäßen Wärmeaustauschkörper gut geführt und durch die großen Nutabstände läßt sich die Leitung leichter aufwickeln. Der große Durchmesser des Wärmeaustauschkörpers erleichtert dabei die Aufwickelbewegung des Bedienungspersonals.

Bei einer weiteren Ausführungsform weist die Nut einen Nutgrund auf, der innerhalb einer Nutöffnung der jeweils einzelnen Nut liegt. Dies hat den Vorteil, daß die Leitung über die unmittelbar auf die Leitung wirkende Hauptkraftkomponente auf den Nutgrund gedrückt werden kann. Diese Nutform ermöglicht auch bei Verengungen oder Hinterschneidungen der Nut gegenüber der Leitung, daß sie ohne erhöhten Kraftaufwand und besondere Geschicklichkeit sicher und paßgerecht am Nutgrund zur Anlage gebracht werden kann.

Die Vorrichtung zum Erwärmen der Flüssigkeit kann durch eine erste und eine zweite Stirnfläche am Wärmeaustauschkörper begrenzt sein. In der ersten Stirnfläche können die Bedienungskomponenten untergebracht sein, sowie ein Display und weitere für den Betrieb der Vorrichtung benötigte Komponenten und in der zweiten Stirnfläche kann die Befestigungsklaue angebracht sein, über die der Wärmeaustauschkörper an einem Ständer befestigt werden kann. Mit dem erfindungsgemäßen Wärmeaustauschkörper ist es also möglich, eine Vorrichtung zu schaffen, die keine weiteren ausladenden Gehäuseteile zur Aufnahme der Betriebskomponenten benötigt. Ein kompaktes Gerät zum Erwärmen von Infusions- und/oder Transfusionslösungen ist zu realisieren.

Aus ergonometrischen Gesichtspunkten ist eine Achse des Wärmeaustauschkörpers im wesentlichen horizontal zu einer Haltevorrichtung, beispielsweise einem Ständer, ausgerichtet. Dabei ist es vorteilhaft, wenn der Wärmeaustauschkörper Mittel zum Drehen und/oder Verschwenken aufweist, wobei insbesondere der Wärmeaustauschkörper an der Haltevorrichtung drehbar und/oder verschwenkbar gehalten ist.

Dies hat den Vorteil, daß mit einfachen Mitteln, nämlich durch ein Drehen des Wärmeaustauschkörpers bzw. durch ein verschwenken des Wärmeaustauschkörpers geprüft werden kann, ob die Leitung über den gesamten Umfang des Wärmeaustauschkörpers auch lagerichtig in der dafür vorgesehenen Nut angeordnet ist. Unbeabsichtigte Störfälle, wie sie bekannterweise durch ein rasches und unter Zeitdruck erfolgtes Einlegen der Leitung in die Nut erzeugt werden, lassen sich mit dem erfindungsgemäßen Wärmeaustauschkörper ausschließen, weil durch eine einfache Drehung und/oder ein Verschwenken des Wärmeaustauschkörpers der gesamte Leitungsverlauf der Leitung eingesehen werden kann.

In einer weiteren Ausgestaltung der Erfindung ist die Nut von einer zweiten Stirnfläche ausgehend zu einer ersten Stirnfläche des Wärmeaustauschkörpers verlaufend als Linksgewinde ausgebildet. Dies hat den Vorteil, daß Rechtshänder die Leitung beginnend von links hinten mit einer kreisenden Rechtsdrehung der Hand, schnell, einfach und sicher in die Nut einlegen können.

Am oder im Wärmeaustauschkörper können Mittel zum Aufwickeln der Leitung vorgesehen sein, wie beispielsweise eine Kurbel oder ein elektrischer Antrieb. Ist der Wärmeaustauschkörper mit einer Kurbel oder einem elektrischen Antrieb ausgestattet, so muß die Leitung in Draufsicht auf das Gerät hinten oben rechts in die erste Nutumschlingung eingedrückt werden und danach wird die Leitung über eine Linksdrehung des gesamten Wärmeaustauschkörpers automatisch in die Nut eingezogen. Dies vereinfacht das Einlegen der Leitung noch weiter.

Sind am oder im Wärmeaustauschkörper Mittel zur Erwärmung einer an der Leitung vorgesehenen Tropfkammer ausgebildet, so wird in einem noch größeren Maße gewährleistet, daß die zu erwärmende Flüssigkeit die vorgegebene Erwärmungstemperatur schnellstmöglich auch bei hohen Durchflußraten erreicht und einer unerwünschten Abkühlung, beispielsweise durch situationsbedingte lange Leitungen, kann effektiv entgegengewirkt werden.

Die Tropfkammer kann dabei in einer Aussparung der ersten oder zweiten Stirnfläche des Wärmeaustauschkörpers lösbar verrastet sein. Dies hat den Vorteil, daß für die Tropfkammer keine eigene Heizeinrichtung geschaffen werden muß und der Leitungsverlauf an der Vorrichtung zum Erwärmen der Flüssigkeit ist nicht gestört.

Es versteht sich, daß die Leitung selbst auch noch isoliert bzw. beheizt werden kann, um unerwünschte Abkühlungseffekte vor und nach der Vorrichtung zum Erwärmen von Flüssigkeiten zu verhindern.

Weitere Vorteile ergeben sich aus der Beschreibung und der beigefügten Zeichnung. Die erwähnten Ausführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen näher erläutert. Es zeigen:

Fig. 1 eine Vorrichtung zum Erwärmen von Flüssigkeiten in perspektivischer Darstellung mit einem im Querschnitt zu einer Achse ellipsenähnlichen Wärmeaustauschkörper;

Fig. 2 einen erfindungsgemäßen Wärmeaustauschkörper, der als gerader Kreiszylinder ausgebildet ist, mit einer Kurbel zum Aufwickeln der Leitung;

Fig. 3 eine Nutform im Querschnitt, mit einem ersten und einem zweiten Nutraum;

Fig. 4 eine weitere Ausführungsform einer Nut im Querschnitt mit einer bogenförmigen Öffnung und mit freien Stegenden, die mit einem wärmeisolierenden Material überzogen sind.

Die einzelnen Figuren der Zeichnung zeigen den erfindungsgemäßen Gegenstand stark schematisiert und sind nicht maßstäblich zu verstehen. Die gegenständlichen Merkmale der einzelnen Figuren sind teilweise stark vergrößert dargestellt, damit ihr Aufbau besser gezeigt werden kann.

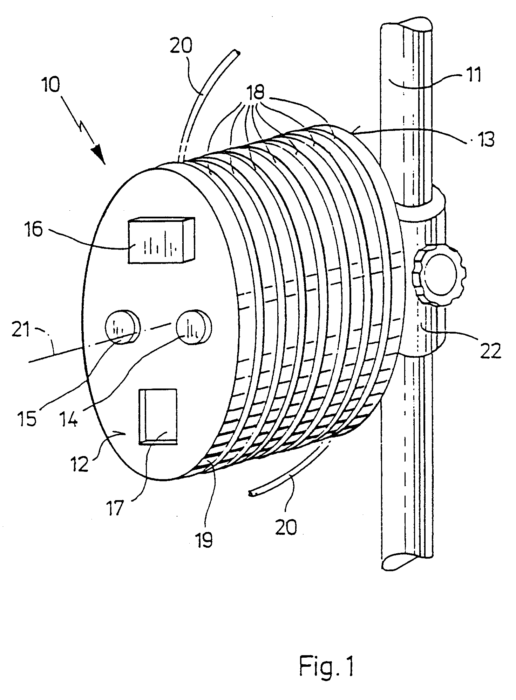

Fig. 1 zeigt einen Wärmeaustauschkörper 10, wie er an einem Ständer 11, beispielsweise einem Infusionsständer, befestigt ist. Der Wärmeaustauschkörper 10 ist von einer ersten Stirnfläche 12 und einer zweiten Stirnfläche 13 begrenzt. Wie die Figur zeigt, ist die erste Stirnfläche 12 als Frontplatte ausgebildet, über die die erfindungsgemäße Vorrichtung bedienbar ist. Auf der Frontplatte ist ein Ein-Ausschalter 14 und ein nicht weiter bezeichneter Wählschalter 15, beispielsweise für die Justierung oder Vorgabe der jeweils gewünschten Temperatur, angeordnet. Mit 16 ist ein Display schematisch angedeutet, das die jeweils gewünschten Werte optisch anzeigt. Eine Aussparung 17 ist an der Frontplatte vorgesehen, in die eine nicht gezeigte Tropf- und/oder Entgasungskammer mit der Option diese zu beheizen, lösbar einrastbar ist. Am Umfang des ellipsenähnlich ausgeführten Wärmeaustauschkörpers 10 sind Nuten 18 ausgebildet, die voneinander über Stege 19 getrennt sind. Die Nuten 18 verlaufen schraubenlinienförmig auf der Außenfläche des Wärmeaustauschkörpers 10. Die Nutumschlingungen verlaufen mit Sicht auf die erste Stirnfläche 12 von der zweiten Stirnfläche 13 ausgehend als Linksgewinde. Eine Leitung 20, die die zu erwärmende Flüssigkeit führt, insbesondere Infusions- und/oder Transfusionslösungen, ist angedeutet. Die Leitung 20 ist in die Nut 18 nicht eingelegt gezeichnet, damit der Wärmeaustauschkörper 10 besser gezeigt werden kann. Die Nut 18 bildet auf der Außenseite des Wärmeaustauschkörpers 10 Windungen mit einer Wärmeübertragungslänge I pro Windung. Die Wärmeübertragungslänge I sowie die gesamte Wärmeübertragungslänge L sind wie folgt definiert

$$I = \pi \cdot d,$$

$$L = \pi \cdot d \cdot w,$$

wobei d dem Durchmesser beim geraden Kreiszylinder minus der 2-fachen Nuttiefe und w der Anzahl der Windungen entspricht. Beim beliebigen zylindrischen Wärmeaustauschkörpers ist d eine Kenngröße, die d beim geraden Kreiszylinder entspricht.

Soll eine Leitung 20 in die Nuten 18 eingelegt werden, so wird ein Leitungsabschnitt L der Leitung 20 in Draufsicht von unten links hinten, mittels einer rechtsdrehenden kreisförmigen Bewegung in die Nuten 18 eingelegt. Die Leitung 20 ist nur abschnittsweise gezeigt. Nach unten führt sie zum Patienten und nach oben führt die Leitung 20 zum Flüssigkeitsbehältnis, in dem die Flüssigkeit gespeichert ist, die in Flußrichtung zum Patienten auf eine bestimmte Temperatur erwärmt werden soll.

Zu einer Achse 21 ist der Wärmeaustauschkörper 10 horizontal ausgerichtet. Wird über eine rechtsdrehende Handbewegung die Kontur des Wärmeaustauschzylinders 10 nachvollzogen, so kann mit dieser Handbewegung die Leitung 20 einfach in die Nuten 18 eingedrückt bzw. kontrolliert werden. Der Einlegevorgang der Leitung beginnt wie schon erwähnt mit Blickrichtung auf die erste Stirnfläche 12 links unten hinten im Bereich der zweiten Stirnfläche 13.

An der zweiten Stirnfläche 13 ist eine Haltevorrichtung 22 vorgesehen, über die der Wärmeaustauschkörper 10 stufenlos verstellbar an einer Stange des Ständers 11 zu befestigen ist.

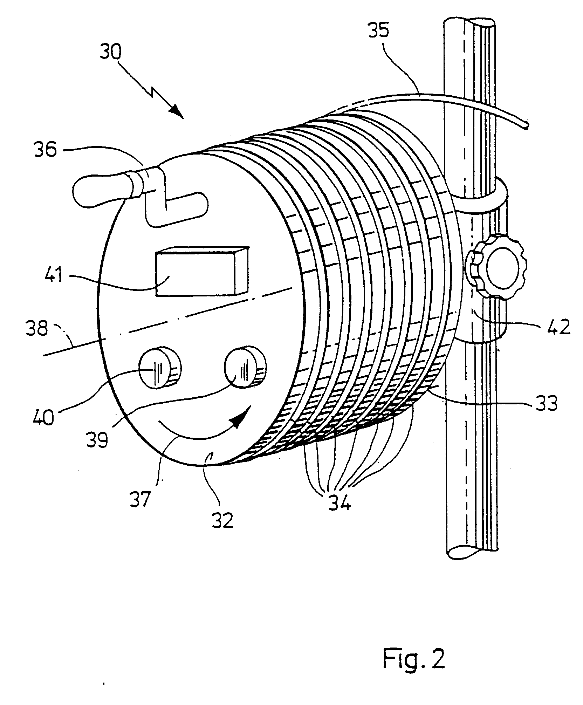

Fig. 2 zeigt einen weiteren erfindungsgemäßen Wärmeaustauschkörper 30, der als gerader Kreishohlzylinder ausgebildet ist. Die Vorrichtung selbst ist durch eine erste und zweite Stirnfläche 32, 33 begrenzt. Auf der Außenseite des Wärmeaustauschkörpers 30 sind Nuten 34 ausgebildet, die eine Leitung 35 unverlierbar im Nutraum halten können. Die Leitung 35 ist formschlüssig in den Nuten 34 gehalten. Wird die Leitung 35 wie in der Figur gezeigt am Wärmeaustauschkörper 30 angelegt und wird über eine Kurbel 36 auf der ersten Stirnfläche 32 der Wärmeaustauschkörper 30 in Pfeilrichtung 37 gedreht, so wickelt sich die Leitung 35 automatisch in die schraubenlinienförmig ausgebildeten Nuten 34 zur ersten Stirnfläche 32 hin. Bei diesem Aufwicklungsvorgang ist der Wärmeaustauschkörper 30 zu einer Achse 38 hin horizontal ausgerichtet. Es ist weiterhin denkbar, daß nach einem Aufwicklungsvorgang der Leitung 35 auf den Wärmeaustauschkörper 30 der Wärmeaustauschkörper 30 in eine andere Positionslage verschwenkt wird.

Auf der ersten Stirnfläche 32 sind mit 39 und 40 Bedienungselemente für die Vorrichtung angedeutet und ein Display 41 ermöglicht die optische Anzeige der Betriebsdaten. Auf der zweiten Stirnfläche 33 ist eine Haltevorrichtung 42 vorgesehen, über die der Wärmeaustauschkörper 30 an dafür vorgesehenen Gegenständen befestigbar ist.

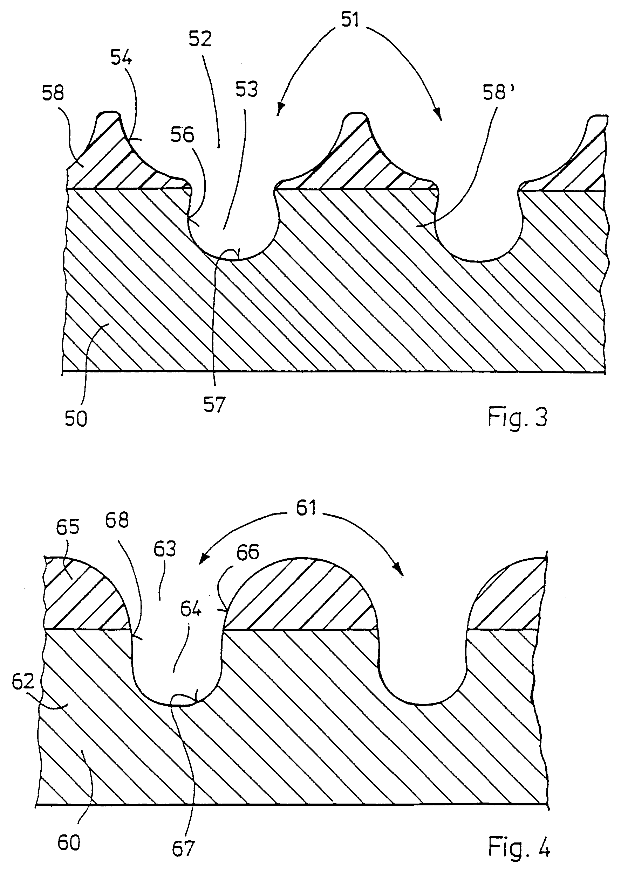

Die Nuten 18 und 34 in den Fig. 1 und 2 sind am Wärmeaustauschkörper 20, 30 nur andeutungsweise gezeigt. Die Fig. 3 und 4 zeigen in vergrößertem Maßstab mögliche bevorzugte Nutformen im Querschnitt, wie sie am Wärmeaustauschkörper 20, 30 ausgebildet sein können.

Fig. 3 zeigt im Querschnitt einen Ausschnitt eines Wärmeaustauschkörpers 50, der aus einem gut wärmeleitfähigen Material gefertigt ist. Die Figur zeigt zwei der schraubenlinienförmig verlaufenden Nuten 51 mit einem ersten Nutraum 52 und einem zweiten Nutraum 53. Der erste Nutraum 52 ist erweitert ausgeführt, so daß ein in den Nutraum 53 einzulegender Schlauch geführt und ohne Hindernisse eingelegt werden kann. Der erste Nutraum 52 weist beispielsweise eine Öffnung auf, die 2,5 Mal größer ist als der Außendurchmesser der in den zweiten Nutraum 53 einzulegenden Leitung. Der erste Nutraum 52 weist Nutflanken 54 auf, die konkav verlaufen. Diese Nutflankenform ist der Finger- bzw. Daumenkuppe angepaßt. Die Nuten 51 sind um ein Vielfaches ihrer Breite voneinander beabstandet, so daß beim Einlegen der Leitung das Überspringen einer Nutumschlingung bestmöglich verhindert wird. Der zweite Nutraum 53 ist von seiner Größe her weitgehend der einzulegenden Leitung angepaßt. Nutflanken 56 verlaufen zum Nutgrund 57 hin mit einem geringfügigen Hinterschnitt, damit die Leitung mit einem verbesserten Wärmekontakt im Nutraum 53 liegt. In Draufsicht fluchten die Nuträume 52, 53. Die jeweiligen Nuten 51 sind durch Stege 58, 58' voneinander getrennt. Die Stege 58 sind aus einem wärmeisolierenden Material gefertigt, das mit einem gut wärmeleitenden Material der Stege 58' des Wärmeaustauschkörpers 50 verbunden ist. Die Stege 58 sind zu den freien Enden hin abgerundet.

Fig. 4 zeigt einen weiteren Ausschnitt eines Wärmeaustauschkörpers 60 mit Nuten 61 im Querschnitt. Die Nuten 61 sind über Stege 62 voneinander getrennt. Ein erster Nutraum 63, dessen Nutflanken konvex verlaufen, geht über in einen zweiten Nutraum 64, der als Haltenut ausgebildet ist. In dem zweiten Nutraun 64 ist die Leitung formschlüssig gehalten, in der die zu erwärmende Flüssigkeit strömt. Der Wärmeaustauschkörper 60 ist aus einem gut wärmeleitfähigen Material gefertigt und die Stegkuppen sind mit einer Beschichtung 65 versehen, die wärmeisolierend wirkt.

Die Vorrichtung zum Erwärmen von Flüssigkeiten weist einen zylindrischen Wärmeaustauschkörper 10 auf, der auf der Außenseite zum Einlegen der von der Flüssigkeit durchströmten Leitung 20 eine Nut 18 aufweist. Die Nut 18 bildet Windungen mit einer Wärmeübertragungslänge l pro Windung. Die Wärmeübertragungslänge l pro Windung ist ausgehend von einer bekannten Wärmeübertragungslänge von 37 cm pro Umschlingung mindestens um einen Faktor 1,4 erhöht. Der Wärmeaustauschzylinder 10 ist im wesentlichen horizontal ausgerichtet.

**Patentansprüche**

1. Medizinische Erwärmvorrichtung von Infusions-, Transfusions- und/oder Spüllösungen, mit einer an einer Leitung (20; 35) für die Lösungen wirksamen regel- und/oder steuerbaren Heizeinrichtung, die einen zylindrischen Wärmeaustauschkörper (10; 30; 50; 60) erwärmt, der auf der Außenseite zum Einlegen der von der Lösung durchströmten Leitung (20; 35) eine Nut (18; 34; 51; 61) aufweist, die Windungen mit einer Wärmeübertragungslänge l pro Windung bildet, dadurch gekennzeichnet, daß

die Wärmeübertragungslänge l pro Windung mindestens 52cm beträgt und daß die Nut (18; 34; 51; 61) im Bereich ihrer Öffnung aus einem wärmeisolierenden Material (58) gefertigt oder mit einem wärmeisolierenden Material (65) beschichtet ist.

2. Medizinische Erwärmvorrichtung nach Anspruch 1, dadurch gekennzeichnet, daß die Öffnung der Nut (51; 61) mindestens 1,6 mal größer ist als der Außendurchmesser der Leitung (20; 35).

3. Medizinische Erwärmvorrichtung nach Anspruch 1 oder 2, dadurch gekennzeichnet, daß sich die Nut (51; 61) bogenförmig öffnet und einen ersten Nutraum (52; 63) mit Nutflanken (54; 66) zum Einlegen der Leitung und einen zweiten Nutraum (53; 64) mit Nutflanken (56; 68) zum Halten der Leitung, eine Haltenut, bildet, wobei die Nutflanken (54; 66) des ersten Nutraums (52; 63) konkav oder konvex oder trichterförmig verlaufen und die Nutflanken (56; 68) des zweiten Nutraums (53; 64) die Leitung formschlüssig umgreifen und gegebenenfalls geringfügig verpressen.

4. Medizinische Erwärmvorrichtung nach einem der Ansprüche 1 bis 3, dadurch gekennzeichnet, daß der Wärmeaustauschkörper (10; 30; 50; 60) im Querschnitt zu einer Achse (21; 38) eine kreisförmige oder ellipsenähnliche Fläche aufspannt.

5. Medizinische Erwärmvorrichtung nach einem der Ansprüche 1 bis 4, dadurch gekennzeichnet , daß der Wärmeaustauschkörper (10; 30; 50; 60) ein Hohlzylinder ist.

6. Medizinische Erwärmvorrichtung nach einem der Ansprüche 1 bis 5, dadurch gekennzeichnet, daß die Nut (18; 34; 51; 61) schraubenlinienförmig verläuft, und daß die Nuten (18; 34; 51; 61) im Querschnitt längs zu einer Achse (21; 38) des Wärmeaustauschkörpers (10; 30; 50; 60) gesehen eine Beabstandung voneinander aufweisen, die größer als die Nutbreite einer Haltenut für die Leitung (20; 35) ist.

7. Medizinische Erwärmvorrichtung nach einem der Ansprüche 1 bis 6, dadurch gekennzeichnet, daß die Nut (51; 61) in Draufsicht einen Nutgrund (57; 67) aufweist, der innerhalb einer Nutöffnung der jeweils einzelnen Nut (51; 61) liegt.

**Claims**

1. Medical warming apparatus for infusion, transfusion, and/or cleansing solutions having a regulatable and/or controllable heating device acting on a conduit (20; 35) for the solution and heating a cylindrical heat exchanging body (10; 30; 50; 60) exhibiting an outer groove (18; 34; 51; 61) for the insertion of a conduit (20; 35) through which the solution is flowing, the groove forming windings with a heat transfer length l per winding, characterized in that the heat transfer length l per winding assumes a value of at least 52 cm and the groove (18; 34; 51; 61) is manufactured in the vicinity of its opening from a heat insulating material (58) or is coated with a heat insulating material (65).

2. Medical warming device according to claim 1, characterized in that the opening of the groove (51; 61) is at least 1.6 times larger than the outer diameter of the conduit (20; 35).

3. Medical warming device according to claim 1 or 2, characterized in that the groove (51; 61) opens in a curved fashion and forms a first groove region (52; 63) with groove sides (54; 66) for the insertion of the conduit and a second groove region (53; 64), i.e. a holding groove, with groove sides (56; 68) for holding the conduit, wherein the groove sides (54; 66) of the first groove region (52; 63) run concave or convex or in a triangular shape and the groove sides (56; 68) of the second groove region (53; 64) tightly surroundingly engage the conduit and, if appropriate, slightly press same.

4. Medical warming device according to one of the claims 1 through 3,characterized in that the heat exchanging body (10; 30; 50; 60) spans a cross section with respect to an axis (21; 38) having a circular or elliptical area.

5. Medical warming device according to one of the claims 1 through 4, characterized in that the heat exchanging body (10; 30; 50; 60) is a hollow cylinder.

6. Medical warming device according to one of the claims 1 through 5, characterized in that the groove (18; 34; 51; 61) runs in a screw-like fashion and the grooves (18; 34; 51; 61) exhibit, in cross section as viewed along an axis (21; 38) of the heat exchanging body (10; 30; 50; 60), a separation from each other which is larger than the groove width of a holding groove for the conduit (20; 35).

7. Medical warming device according to one of the claims 1 through 6, characterized in that the groove (51; 61) exhibits, in plan view, a groove bottom (57; 67) which lies within a groove opening of each individual groove (51; 61).

**Revendications**

1. Dispositif médical de chauffage de solutions pour perfusions, transfusions et/ou lavages, comportant un organe de chauffage qui agit sur une conduite (20; 35) pour les solutions, peut être réglé et/ou commandé et chauffe un appareil cylindrique (10; 30; 50; 60) d'échange de chaleur qui présente sur sa face extérieure, pour y placer la conduite (20; 35) parcourue par la solution, une rainure (18; 34; 51; 61) qui forme des spires d'une longueur l de transmission de la chaleur par spire, caractérisé par le fait que la longueur l de transmission de chaleur par spire vaut au moins 52cm et que la rainure (18; 34; 51; 61), dans la zone de son ouverture, est fabriquée en un matériau thermiquement isolant (58) ou est revêtue d'un matériau thermiquement isolant (65).

2. Dispositif médical de chauffage selon la revendication 1, caractérisé par le fait que l'ouverture de la rainure (51; 61) est au moins 1,6 fois plus grande que le diamètre extérieur de la conduite (20; 35).

3. Dispositif médical de chauffage selon la revendication 1 ou 2, caractérisé par le fait que la rainure (51; 61) s'ouvre en forme d'arc et forme un premier espace de rainure (52; 63) avec des flancs (54; 66) de rainure pour introduire la conduite et un second espace de rainure (53; 64) avec des flancs (56; 68) pour maintenir la conduite, une rainure de maintien, les flancs (54; 66) du premier espace de rainure (52; 63) étant d'allure concave ou convexe ou en forme d'entonnoir et les flancs (56; 68) du second espace de rainure (53; 64) enserrant la conduite par complémentarité de forme et la pressant éventuellement légèrement.

4. Dispositif médical de chauffage selon l'une des revendications 1 à 3, caractérisé par le fait que l'appareil d'échange de chaleur (10; 30; 50; 60) définit, en coupe par rapport à un axe (21; 38), une surface de forme circulaire ou semblable à une ellipse.

5. Dispositif médical de chauffage selon l'une des revendications 1 à 4, caractérisé par le fait que l'appareil d'échange de chaleur (10; 30; 50; 60) est un cylindre creux.

6. Dispositif médical de chauffage selon l'une des revendications 1 à 5, caractérisé par le fait que la rainure (18; 34; 51; 61) a l'allure d'une hélice et que, vues longitudinalement par rapport à un axe (21; 38) de l'appareil d'échange de chaleur (10; 30; 50; 60), les rainures (18; 34; 51; 61) présentent, en coupe, une distance l'une de l'autre qui est supérieure à la largeur d'une rainure de maintien pour la conduite (20; 35).

7. Dispositif médical de chauffage selon l'une des revendications 1 à 6, caractérisé par le fait que la rainure (51; 61) présente, en vue de dessus, un fond de rainure (57; 67) qui se situe à l'intérieur d'une ouverture de chaque rainure respective (51; 61).

Fig. 1

Fig. 2

Fig. 3

Fig. 4